# EUROPÄISCHE PATENTSCHRIFT

(11) **EP 3 324 199 B1**
(45) Veröffentlichungstag und Bekanntmachung des Hinweises auf die Patenterteilung: **03.11.2021**
(21) Anmeldenummer: 17181239.9
(22) Anmeldetag: 13.07.2017
(51) Int. Cl.: G01R 33/561, G01R 33/563

(54) **BSSFP-MR-ANGIOGRAPHIE MIT VERWENDUNG EINES HF-PULSES MIT RAMPENFÖRMIGEM ANREGUNGSPROFIL**
BSSFP MR ANGIOGRAPHY USING AN RF PULSE HAVING A RAMP-SHAPED EXCITATION PROFILE
ANGIOGRAPHIE PAR IRM UTILISANT UNE SÉQUENCE EN PRÉCESSION LIBRE EN RÉGIME PERMANENT ÉQUILIBRÉ (BSSFP) AVEC UNE IMPULSION RF AYANT UN PROFIL D'EXCITATION EN FORME DE RAMPE

(43) Veröffentlichungstag der Anmeldung: 23.05.2018
(73) Patentinhaber: Siemens Healthcare GmbH, 91052 Erlangen (DE)
(72) Erfinder: Schmitt, Peter, 91085 Weisendorf (DE); Speier, Peter, 91056 Erlangen (DE)

(56) Entgegenhaltungen:
- US-A- 5 307 014
- OPPELT A ET AL: "FISP: EINE NEUE SCHNELLE PULSSEQUENZ FUER DIE KERNSPINTOMOGRAPHIE//FISP-A NEW FAST MRI SEQUENCE", ELECTROMEDICA, SIEMENS AG MEDICAL ENGINEERING GROUP, ERLANGEN, DE, Bd. 54, Nr. 1, 1. Januar 1986 (1986-01-01) , Seiten 15-18, XP012046413, ISSN: 0013-4724, DOI: 10.1063/1.370589
- NAGELE T ET AL: "THE EFFECTS OF LINEARLY INCREASING FLIP ANGLES ON 3D INFLOW MR ANGIOGRAPHY", MAGNETIC RESONANCE IN MEDICINE, JOHN WILEY & SONS, INC, US, Bd. 31, Nr. 5, 1. Mai 1994 (1994-05-01), Seiten 561-566, XP000443553, ISSN: 0740-3194
- ZUR Y: "Optimized Slab Profile for 3D-TOF Angiography", PROCEEDINGS OF THE SOCIETY OF MAGNETIC RESONANCE IN MEDICINE, BERKELEY, SMR, US, Bd. 2, 6. August 1994 (1994-08-06), Seite 960, XP002207497, ISSN: 1065-9889

## Beschreibung

Die Erfindung betrifft ein Verfahren zur Aufnahme von Magnetresonanzdaten aus einem aufzunehmenden Bildgebungsvolumen eines Patienten, in dem Flüssigkeit, insbesondere Blut, bewegt ist, mittels einer bSSFP-Magnetresonanzsequenz, bei der innerhalb des Bildgebungsvolumens befindliche Spins mittels eines Hochfrequenzpulses zyklisch angeregt werden, mit einer Magnetresonanzeinrichtung. Daneben betrifft die Erfindung eine Magnetresonanzeinrichtung, ein Computerprogramm und einen elektronisch lesbaren Datenträger.

Eine Aufgabe, die sich in der klinischen Magnetresonanzbildgebung oft stellt, ist die Bildgebung von Blutgefäßen beziehungsweise des darin befindlichen Blutes. Hierzu wurden im Stand der Technik bereits einige Möglichkeiten zur Aufnahme entsprechender Ziel-Bildgebungsvolumina/Schichten vorgeschlagen.

Es sind bereits Methoden zur Magnetresonanzbildgebung bekannt, in denen Nicht-Gleichgewichtszustände genutzt werden, um die Magnetresonanzdaten aufzunehmen. Dabei ist die Magnetisierung in einer Schicht, im Allgemeinen also in einem Bildgebungsvolumen, nicht konstant, während das Bild aufgenommen wird, sondern wird letztlich während der Akquisition "aufgebraucht". Während die Bildkodierung fortschreitet, wird das kodierte Magnetresonanzsignal zunehmend gesättigt, bis ein Gleichgewichtszustandswert erreicht wird, der typisch für die Bildgebungstechnik ist. Die Rate, mit dem das Magnetresonanzsignal hin zu dem Gleichgewichtszustand zerfällt, (im Folgenden Zerfallsrate) hängt von den Relaxationsparametern und der Bildgebungstechnik ab.

Liegt nun, wie im Fall bewegten Blutes, ein makroskopischer räumlicher Transport von Material und somit Magnetisierung während der Bildgebung vor, ist der Sättigungszustand grob korreliert mit der räumlichen Position. Das höchste Magnetresonanzsignal wird erhalten, wo der Fluss in die Schicht eintritt, das niedrigste Magnetresonanzsignal wird am anderen Ende der Schicht erhalten. Die Zerfallsrate hinsichtlich der Gleichgewichtszustände lässt sich für verschiedene Bildgebungstechniken ermitteln, beispielsweise für die FLASH-Bildgebungstechnik bei T1>TR als Funktion des Flipwinkels pro Zeit (FLASH = Fast Low-Angle Shot). Für die bSSFP-Bildgebungstechnik (Balanced Steady-State Free Precession - balancierte Bildgebung mit freier Präzession im Gleichgewichtszustand) ergibt sich, dass die Zerfallsrate eine

Flipwinkel-abhängige Mischung aus T1 und T2 ist, vgl. hierzu den Artikel von Patrick Le Roux, "Simplified model and stabilization of SSFP sequences", Journal of Magnetic Resonance 163 (2003), Seiten 23 bis 37.

Die Zerfallsrate (häufig mit R bezeichnet) ist hochrelevant, wenn das Ziel der Bildgebung die Vermessung des bewegten Materials, konkret insbesondere fließenden Blutes zur Aufnahme der Blutgefäße, ist, da die Zerfallsrate in den Gleichgewichtszustand dann bestimmt, wie lange das bewegte Material von dem statischen Hintergrund getrennt werden kann und wie weit es sich bewegt hat. Für die Magnetresonanz-Angiographie stellt dies mithin eine Begrenzung dafür dar, wie weit ein sich aufweitender Gefäßbaum visualisiert werden kann.

bSSFP-basierte Time-of-Flight-Messungen (ToF-Messungen) sind in dieser Hinsicht besonders geeignet für Magnetresonanzeinrichtungen mit niedrigem Grundmagnetfeld, nachdem die standardisierte, gradientenechobasierte TOF-Messung bezüglich des Signal-zu-Rauschverhältnisses (SNR) ohnehin nachteilig ist. bSSFP-Bildgebungstechniken profitieren jedoch von einem erhöhten T2/T1-Verhältnis des Blutes, SAR-Reduzierung und für Spitzen-Bl-Feld (Hochfrequenzfeld) sowie B0-Feld (Grundfeld) verbesserter Homogenität. Die eher nachteiligen Aspekte der bSSFP-Bildgebungstechniken sind bei niedrigeren Grundfeldern weniger relevant.

Um den Zerfall innerhalb des Bildgebungsvolumens, also der Schicht, zu minimieren, wurde vorgeschlagen, das Bildgebungsvolumen in eine Vielzahl von Subvolumen ("Slabs") mit reduzierter Dicke in der Flussrichtung der Flüssigkeit zu unterteilen, was jedoch die Effizienz reduziert, da jedes Subvolumen zur Stabilisierung des Hintergrundkontrastes einen Vorabscan benötigt, für jeden Slab überabgetastet werden muss und das Signal-zu-Rausch-Verhältnis wenigstens für das Hintergrundgewebe reduziert wird.

Damit ein Gewebe hell in der FLASH-Bildgebungstechnik dargestellt wird, sind der Flipwinkel und die Repetitionszeit TR so zu wählen, dass das Inverse der Zerfallsrate in den Gleichgewichtszustand größer als die T1-Relaxationszeit des Gewebes/der Flüssigkeit ist. In Anwendungen wie der TOF-Bildgebung kann dies für Blut nicht erreicht werden, nachdem die erforderlichen Flipwinkel für hinreichende Hintergrundunterdrückung in einer inversen Zerfallsrate resultieren, die kleiner als die T1-Relaxationszeit für Blut ist. Das bedeutet, dass das Blutsignal zerfällt, während sich das Blut durch das Bildgebungsvolumen bewegt. Ein typisches Beispiel für einen solchen Fall ist bei einem Flipwinkel von 15° und einer TR von 25 ms gegeben, da sich dann als Inverses der Zerfallsrate 720 ms ergeben.

Um dieses Problem zu lösen, wurde für die FLASH-Bildgebungstechnik vorgeschlagen, Hochfrequenzpulse zu verwenden, die einen sich verändernden Flipwinkel über das Bildgebungsvolumen generieren, welche als Rampenpulse beziehungsweise TONE-Pulse ("Tilt-Optimized Nonsaturated Excitation"-Pulse) bezeichnet werden. An der Schichtgrenze, wo die Flüssigkeit, insbesondere das Blut, in das Bildgebungsvolumen einströmt, wird ein reduzierter Flipwinkel verwendet. Typischerweise variiert der Flipwinkel linear über das Bildgebungsvolumen, bis er den maximalen Flipwinkel auf der gegenüberliegenden Seite beziehungsweise Schichtgrenze erreicht. Die verwendeten Rampenpulse sind stark asymmetrisch in der Zeit, um die Echozeit und die SAR zu reduzieren. Ein niedrigerer Flipwinkel auf der Einströmseite führt zu einer deutlich längeren Zeitdauer, bis der Gleichgewichtszustand erreicht wird, mithin zu einer niedrigeren Zerfallsrate. Im oben genannten Beispiel kann unter Verwendung von Rampenpulsen dann, wenn mit dem halben maximalen Flipwinkel auf der Einströmseite begonnen wird, eine inverse Zerfallsrate von 3340 ms erreicht werden, die deutlich größer als die T1-Relaxationszeit von Blut ist.

Soll die bSSFP-Bildgebungstechnik zur Aufnahme des Blutflusses verwendet werden, werden üblicherweise Verfahren eingesetzt, deren Fokus auf der Bildgebung hoher Flussgeschwindigkeiten liegt. Die Sequenz wird beispielsweise unterbrochen, solange der Fluss nicht gesättigtes Material in das Bildgebungsvolumen einbringt, wonach das Bildgebungsvolumen schnell aufgenommen wird, während der Zerfall innerhalb des Bildgebungsvolumens vernachlässigbar ist. Ein zweidimensionales Beispiel ist die QISS-Technik, worin die Sequenz gestoppt wird, während das Herz frisches Blut in das Bildgebungsvolumen drückt. Hierzu sei beispielsweise auf den Artikel von R. R. Edelman et al., "Quiescent-interval single-shot unenhanced magnetic resonance angiography of peripheral vascular disease: Technical considerations and clinical feasibility", Magnetic Resonance in Medicine 63 (2010), Seiten 951 - 958, verwiesen.

Wenn ein langsamerer Fluss aufgenommen werden soll, kann der Flipwinkel reduziert werden, was die Zerfallsrate erniedrigt, indem der T1-Anteil erhöht und der T2-Anteil erniedrigt wird, nachdem üblicherweise T1>T2 gilt. Ein Beispiel für eine dynamische, nicht kontrastmittelverstärkte Angiographie unter Verwendung der bSSFP-Bildgebungstechnik mit kontinuierlicher Aufnahme ist beispielsweise in einem Artikel von X. Bi et al., "Non-contrast-enhanced four-dimensional (4D) intracranial MR angiography: a feasibility study", Magnetic Resonance in Medicine 63 (2010), Seiten 835 - 841, beschrieben.

Nachteilhaft bei der bekannten Nutzung von bSSFP-Bildgebungstechniken zur Bildgebung bewegter Flüssigkeit, insbesondere von Blut, ist, dass aufgrund der verschiedenen Gleichgewichtszustände der in das Bildgebungsvolumen transportierten Flüssigkeit ein ungleicher Kontrast gegeben ist, beziehungsweise aufgrund zu starker Flipwinkelerniedrigung zu große Signalverluste entlang der Bildgebungsstrecke auftreten können.

US 5 307 014 betrifft Inflow-MR-Angiographie mit räumlich variierenden Flipwinkeln. Dort wird vorgeschlagen, in der Volumeneingangsebene einen relativ geringen Flipwinkel zu verwenden, der monoton durch das Volumen in Flussrichtung ansteigt.

Der Artikel von A. Oppelt et al., "FISP: eine neue schnelle Pulssequenz für die Kernspintomographie", electromedica 54 (1986), Seiten 15 bis 18, beschreibt die auch als SSFP-Sequenz bezeichnete FISP-Sequenz. Dabei werden die Gradienten so geschaltet, dass die Phase der Quermagnetisierung nach einem HF-Puls und vor dem nächsten Puls nicht verändert wird.

Der Erfindung liegt die Aufgabe zugrunde, eine Möglichkeit zur Nutzung der bSSFP-Bildgebungstechnik anzugeben, die einerseits den visualisierten Abstand von der Einströmseite des Bildgebungsvolumens maximiert und andererseits eine hinreichende Signalstärke über das gesamte Bildgebungsvolumen erlaubt.

Zur Lösung dieser Aufgabe sind erfindungsgemäß ein Verfahren mit den Merkmalen des Anspruchs 1, eine Magnetresonanzeinrichtung mit den Merkmalen des Anspruchs 8, ein Computerprogramm mit den Merkmalen des Anspruchs 9 und ein elektronisch lesbarer Datenträger mit den Merkmalen des Anspruchs 10 vorgesehen. Vorteilhafte Weiterbildungen ergeben sich aus den Unteransprüchen.

Bei einem erfindungsgemäßen Verfahren zur Aufnahme von Magnetresonanzdaten aus einem aufzunehmenden Bildgebungsvolumen, insbesondere einer Schicht, eines Patienten, in dem Flüssigkeit, insbesondere Blut, so bewegt ist, dass die Flüssigkeit auf einer Einströmseite in das Bildgebungsvolumen einströmt und auf einer gegenüberliegenden Ausströmseite aus dem Bildgebungsvolumen ausströmt, wobei die Aufnahme der Magnetresonanzdaten mit einer Magnetresonanzeinrichtung und mittels einer bSSFP-Magnetresonanzsequenz erfolgt, bei der innerhalb des Bildgebungsvolumens befindliche Spins mittels eines Hochfrequenzpulses zyklisch angeregt werden, ist vorgesehen, dass als Hochfrequenzpuls ein einen räumlich innerhalb des Bildgebungsvolumens veränderlichen Flipwinkel der Spins einstellender, insbesondere zeitlich symmetrischer, Rampenpuls verwendet wird, wobei der Flipwinkel auf der Einströmseite, von der die Flüssigkeit in das Bildgebungsvolumen einströmt, niedriger gewählt wird als auf der Ausströmseite, auf der die Flüssigkeit ausströmt, und monoton ansteigt.

Erfindungsgemäß wird mithin vorgeschlagen, eine kontinuierliche bSSFP-Bildaufnahme, insbesondere für schnellen Fluss, zu realisieren, indem der üblicherweise bei bSSFP-Magnetresonanzsequenzen verwendete SINC-Hochfrequenzpuls durch einen speziell für die bSSFP-Magnetresonanzsequenz optimierten Rampenpuls (TONE-Puls) ersetzt wird. Wie bei der oben diskutierten FLASH-Magnetresonanzsequenz sorgt der Flipwinkelverlauf dafür, dass die verstärkten Magnetresonanzsignale einfließender Flüssigkeit längere Zeit erhalten bleiben, indem die Zerfallsrate in den Gleichgewichtszustand an der Grenze des Bildgebungsvolumens, wo der Fluss eintritt, minimiert wird, und das Magnetresonanzsignal auf der anderen Seite beziehungsweise an der anderen Grenze des Bildgebungsvolumens, der Ausströmseite, maximiert wird. Der zugrundeliegende Mechanismus entspricht nicht dem bei der FLASH-Magnetresonanzbildgebung, sondern hängt von der Tatsache ab, dass die T1-Relaxationszeit größer als die T2-Relaxationszeit ist, was bei Blut zutrifft, wie oben dargelegt wurde.

Der Rampenpuls ist bevorzugt in der Zeit symmetrisch zu wählen, was die Integration in das balancierte Gradientenschema der bSSFP-Magnetresonanzsequenz verbessert, mithin keine zusätzlichen Momente einbringt. Die symmetrische Ausgestaltung des Rampenpulses hat den weiteren Vorteil, dass die Phasendrift über das Bildgebungsvolumen minimiert wird, die nachteilhaft für das Gleichgewichtszustands-Magnetresonanzsignal schnell fließenden Materials wäre, nachdem nachfolgende Hochfrequenzpulse (also Rampenpulse) die schnellfließenden Spins an verschiedenen Positionen beeinflussen würden, mithin mit unterschiedlichen Phasen. Insgesamt wird also eine Optimierung der bSSFP-Magnetresonanztechnik durch Verwendung angepasster Rampenpulse (TONE-Pulse) vorgeschlagen, die insbesondere für 3D-TOFartige Anwendungen geeignet ist. Das Ermöglichen der Nutzung der bSSFP-Magnetresonanzsequenz statt der FLASH-Magnetresonanzsequenz für TOF-Anwendungen resultiert in deutlich höherem Signal-zu-Rauschverhältnis und schnelleren Kodierungszeiten aufgrund kürzerer Repetitionszeiten (TR), so dass für klinische Anwendungen geeignete, nicht kontrastmittelverstärkte Angiographie insbesondere auch auf Magnetresonanzeinrichtungen mit niedrigem Grundfeld ermöglicht wird.

Konkret kann vorgesehen sein, dass der monoton steigende Flipwinkelverlauf innerhalb des Bildgebungsvolumens zur Homogenisierung der Magnetresonanzdaten der Flüssigkeit innerhalb des Bildgebungsvolumens und/oder linear steigend von der Einströmseite zu der Ausströmseite gewählt wird. Ist die Flussgeschwindigkeit durch das Bildgebungsvolumen, insbesondere die Schicht bekannt, lassen sich geeignete Zerfallsraten und mithin Flipwinkel ermitteln, die dem Rampenpuls als Flipwinkelverlauf zugrunde gelegt werden können. Die entsprechenden Zusammenhänge sind, wie eingangs dargelegt wurde, im Stand der Technik bereits bekannt bzw. experimentell und/oder theoretisch ermittelbar. Diese Ausgestaltung ermöglicht ein verbessertes Erscheinungsbild von aus aufgenommenen Magnetresonanzdaten abgeleiteten Magnetresonanzbildern. Der Flipwinkelverlauf steigt erfindungsgemäß monoton und ohne Sprünge, mithin glatt, von der Grenze auf der Einströmseite zu der Grenze auf der Ausströmseite an. Dabei kann eine klassische, lineare Rampe gewählt werden, jedoch sind auch andere geeignete Flipwinkelverläufe möglich, beispielsweise nichtlineare, insbesondere konkave, Verläufe, beispielsweise quadratische Verläufe.

Zweckmäßigerweise wird bei der Auslegung des Rampenpulses als Randbedingung eine maximale Belastbarkeit eines Hochfrequenzverstärkers der Magnetresonanzeinrichtung und/oder bei der Auslegung des Gesamtaufnahmevorgangs ein einzuhaltender SAR-Grenzwert berücksichtigt. Die bSSFP-Bildgebungstechnik erfordert üblicherweise höhere Flipwinkel, beispielsweise im Bereich von 50 bis 70 Grad, so dass überprüft werden sollte, ob SAR-Grenzwerte und/oder die maximale Belastbarkeit von Hochfrequenzverstärkern eingehalten werden. Die SAR-Effizienz sollte also hinreichend klein gehalten werden, was Leistungsintegrale/Amplitutenintegrale angeht, während gleichzeitig das maximal erforderliche B1-Feld klein genug gehalten werden soll, um es durch die Hochfrequenzverstärker bereitstellen zu können.

Gemäß der vorliegenden Erfindung ist vorgesehen, dass eine maximale Steigung des Flipwinkels benachbart zu der die Einströmseite bildenden Grenze der Schicht durch einen Grenzwert beschränkt wird, der anhand der Repetitionszeit der bSSFP-Magnetresonanzsequenz und der Flussgeschwindigkeit der Flüssigkeit ermittelt wird. Insbesondere kann dabei vorgesehen sein, dass das Produkt der Flussgeschwindigkeit der Flüssigkeit, der Repetitionszeit und der maximalen Steigung (Flipwinkelgradient) kleiner als der maximal einzustellende Flipwinkelwert und/oder kleiner als ein fester Grenzwert in einem Intervall von 1,5° bis 15°, beispielsweise 10°, gewählt wird. Besonders zweckmäßig ist es dabei, wenn der Flipwinkelverlauf auf der Einströmseite außerhalb der Schicht bei einem Startwert kleiner als 5°, insbesondere 0°, beginnend, insbesondere linear, bis zur Ausströmseite der Schicht ansteigt, insbesondere unter Verwendung einer konstanten Steigung. Der Gedanke hierbei ist, dass bei der bSSFP-Magnetresonanzsequenz keine starken Sprünge zwischen räumlich bzw. zeitlich benachbarten Spins auftreten sollten, was die Flipwinkel angeht. Der bereits außerhalb des Bildgebungsvolumens insbesondere nahe bei Null beginnende Flipwinkelverlauf, der nicht zu stark ansteigt, wirkt als eine Art Gleichgewichts-Katalysator für einfließende Flüssigkeit, insbesondere einfließendes Blut. Die initiale Hochramp-Steigung auf der Einströmseite hängt von der maximal erwarteten Flussgeschwindigkeit ab. Die Spins der einströmenden Flüssigkeit werden so bereits vor dem Eintritt in das Bildgebungsvolumen adäquat vorbereitet, um das geeignete Signal liefern zu können.

Allgemein kann die durch den Flipwinkelverlauf gebildete Rampe von der Grenze des Bildgebungsvolumens auf der Einströmseite zu der Grenze des Bildgebungsvolumens auf der Ausströmseite für eher langsame Flussgeschwindigkeiten über 10 % des Nenn-Flipwinkels, der auf der Grenze an der Ausströmseite erreicht ist, abdecken, beispielsweise 50 %, wie dies häufig auch bei FLASH-Magnetresonanzsequenzen vorgenommen wird. Hierzu ist zweckmäßigerweise, wie soeben dargelegt, darauf zu achten, dass das initiale Hochrampen außerhalb des Bildgebungsvolumens (also insbesondere in einem Oversampling-Bereich) hinreichend flach ist, um die gewünschten Bildgebungseigenschaften für die bSSFP-Magnetresonanzsequenz zu erreichen. Für hohe Flussgeschwindigkeiten hat sich gezeigt, dass eine Abweichung von einem linearen Flipwinkelverlauf hin zu einer konkaven Form, beispielsweise einer quadratischen Rampe, vorteilhaft sein kann, um die Robustheit zu erhöhen. Der zeitliche Pulsverlauf des Rampenpulses kann, wie grundsätzlich bekannt, durch Fouriertransformation des vorgegebenen Flipwinkelverlaufs im Frequenzraum ermittelt werden. Ein Verfahren zur Ermittlung des Rampenpulses kann mithin umfassen, zunächst einen realwertigen Flipwinkelverlauf im Frequenzraum zu ermitteln beziehungsweise festzulegen, wobei hier beispielsweise experimentell durch Messungen bei unterschiedlichen Flussgeschwindigkeiten Flipwinkelverläufe bestimmt werden können und/oder aus Experimenten und/oder theoretischen Berechnungen abgeleitete mathematische Zusammenhänge herangezogen werden können. Der Flipwinkelverlauf wird dann in die Zeitdomäne fouriertransformiert, um eine komplexwertige Rohpulsform zu erhalten.

Eine vorteilhafte Weiterbildung der Erfindung sieht vor, dass der Pulsverlauf durch Anwendung eines Tiefpassfilters, insbesondere eines Hannfilters, geglättet wird. Die finale Pulsform mit begrenzter Zahl an Stützstellen kann dann durch Anwendung eines Tiefpassfilters auf die Rohpulsform in der Zeitdomäne ermittelt werden, insbesondere durch Anwendung eines Hann-Filters.

Neben dem Verfahren betrifft die Erfindung auch eine Magnetresonanzeinrichtung, aufweisend eine zur Durchführung des erfindungsgemäßen Verfahrens ausgebildete Steuereinrichtung. Sämtliche Ausführungen bezüglich des erfindungsgemäßen Verfahrens lassen sich analog auf die erfindungsgemäße Magnetresonanzeinrichtung übertragen, mit welcher mithin ebenso die bereits genannten Vorteile erhalten werden können. Neben der üblicherweise in Magnetresonanzeinrichtungen vorgesehenen Sequenzsteuereinheit, die den Ablauf der Magnetresonanzsequenz durch Ansteuerung der entsprechenden weiteren Komponenten der Magnetresonanzeinrichtung koordiniert, kann eine derartige Steuereinrichtung mithin eine Pulsermittlungseinheit zur Ermittlung eines Rampenpulses, wie beschrieben, aufweisen, insbesondere, wenn wenigstens eine Abschätzung der Flussgeschwindigkeit der Flüssigkeit, insbesondere des Blutes, vorliegt.

Ein erfindungsgemäßes Computerprogramm ist beispielsweise direkt in einen Speicher einer Steuereinrichtung einer Magnetresonanzeinrichtung ladbar und weist Programmmittel auf, um die Schritte eines hierin beschriebenen Verfahrens auszuführen, wenn das Computerprogramm in der Steuereinrichtung der Magnetresonanzeinrichtung ausgeführt wird. Das Computerprogramm kann auf einem erfindungsgemäß elektronisch lesbaren Datenträger gespeichert sein, welcher mithin darauf gespeicherte elektronisch lesbare Steuerinformationen umfasst, welche zumindest ein genanntes Computerprogramm umfassen und derart ausgestaltet sind, dass sie bei Verwendung des Datenträgers in einer Steuereinrichtung einer Magnetresonanzeinrichtung ein hierin beschriebenes Verfahren durchführen. Bei dem Datenträger kann es sich um einen nichttransienten Datenträger, insbesondere eine CD-ROM handeln.

Weitere Vorteile und Einzelheiten der vorliegenden Erfindung ergeben sich aus den im Folgenden beschriebenen Ausführungsbeispielen sowie anhand der Zeichnung. Dabei zeigen:
- Fig. 1: ein von einem Blutgefäß durchflossenes Bildgebungsvolumen mit einem Flipwinkelverlauf,
- Fig. 2: Graphen zu einer Ausgestaltung und Ermittlung eines Rampenpulses für eine bSSFP-Magnetresonanzsequenz, und
- Fig. 3: eine Prinzipskizze einer erfindungsgemäßen Magnetresonanzeinrichtung.

Soll in einem Bildgebungsvolumen, beispielsweise einer Schicht, eine dreidimensionale TOF-Bildgebung fließenden Blutes in einem Zielgebiet eines Patienten erfolgen, beispielsweise für eine Magnetresonanz-Angiographie, wird in Ausführungsbeispielen der vorliegenden Erfindung eine bSSFP-Magnetresonanzsequenz verwendet, die bekanntermaßen regelmäßige Hochfrequenzpulse nutzt, um die in Phase bewegten Spins im Bildgebungsvolumen mit der Zeit in einen Gleichgewichtszustand (steady state) zu bringen. Statt einem SINC-Hochfrequenzpuls wird vorgeschlagen, einen Rampenpuls als Hochfrequenzpuls zu verwenden, um auf der Einströmseite durch Verwendung niedriger Flipwinkel eine niedrigere Zerfallsrate in den Gleichgewichtszustand zu erzielen, auf der Ausströmseite dennoch ein hinreichend hohes Signal zu halten.

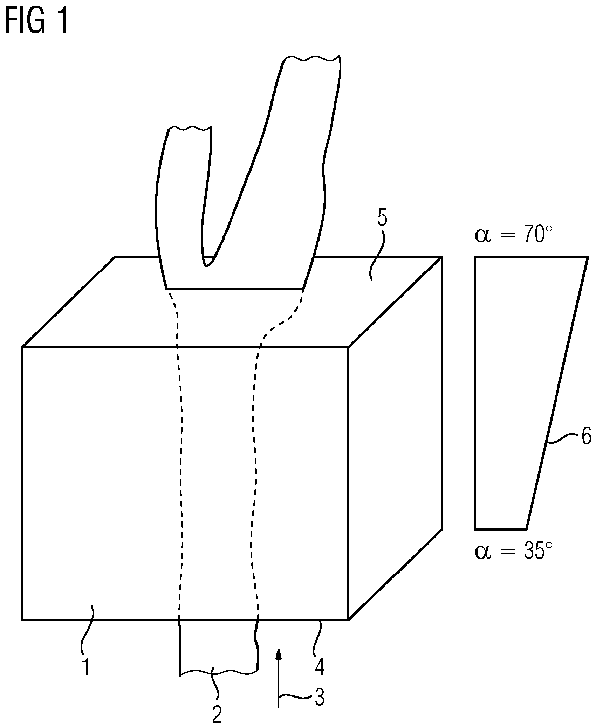

Fig. 1 erläutert dies in Form einer vereinfachten Prinzipskizze eines dreidimensionalen Bildgebungsvolumens 1, durch das sich ein Blutgefäß 2 erstreckt, genauer. Der Pfeil 3 symbolisiert dabei die Flussrichtung, so dass das Bildgebungsvolumen 1 eine Einströmseite 4 und eine Ausströmseite 5 aufweist.

Der Kasten 6 deutet den Flipwinkelverlauf innerhalb des Bildgebungsvolumens 1 von der Grenze des Bildgebungsvolumens 1 an der Einströmseite 4 zu der Grenze des Bildgebungsvolumens 1 an der Ausströmseite 5 an. Der Nenn-Flipwinkelwert der bSSFP-Magnetresonanzsequenz ist vorliegend 70°, allerdings wird auf der Einströmseite 4 zunächst mit einem Flipwinkel von 35° begonnen. Der Rampenpuls ist so gestaltet, dass der Flipwinkel linear bis zum Erreichen von 70° an der Grenze der Ausströmseite 5 ansteigt. Damit wird erreicht, dass die Zerfallsrate in den Gleichgewichtszustand für in das Bildgebungsvolumen 1 eintretende Spins des Blutes geringer ist, es mithin länger dauert, bis diese Spins den Gleichgewichtszustand erreichen, so dass sie insbesondere auf ihrem gesamten Weg durch das Bildgebungsvolumen 1 ein erhöhtes Magnetresonanzsignal liefern, was beispielsweise für eine klare Erkennbarkeit des Blutgefäßes 2 sorgt. Der ansteigende Flipwinkel zur Ausströmseite 5 sorgt dafür, dass dort hinreichende Signalstärke vorliegt.

Das in Fig. 1 gezeigte Beispiel ist dabei insbesondere für langsamen Blutfluss geeignet, während es für starken Blutfluss mit hohen Flussgeschwindigkeiten auch denkbar ist, andere Randwerte für den Flipwinkel beziehungsweise andere, bevorzugt konkave, Verlaufsformen, insbesondere konkave, beispielsweise quadratische, Verläufe, einzusetzen.

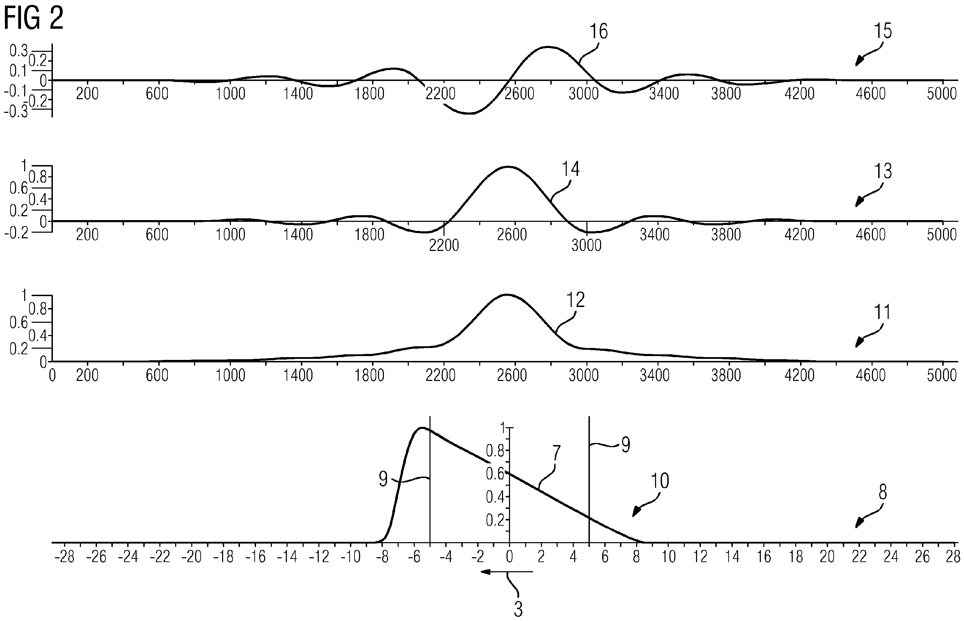

Fig. 2 zeigt einen Ausgangs-Flipwinkelverlauf und die resultierende Pulsform (zeitlicher Pulsverlauf) eines beispielhaften Rampenpulses in Form mehrerer Graphen genauer. Ausgangspunkt zum Ermitteln eines geeigneten Rampenpulses ist ein vorzugebender Flipwinkelverlauf 7 im Frequenzraum, vgl. Graph 8. Die Linien 9 geben dabei die Begrenzung des Bildgebungsvolumens 1 im Frequenzraum an. Ersichtlich ist innerhalb des Bildgebungsvolumens 1 ein linearer, monoton steigender, glatter Verlauf des Flipwinkels von der Einströmseite 4 zur Ausströmseite 5 vorgesehen, der nach Erreichen der Grenze der Ausströmseite, so schnell die Begrenzungen, beispielsweise der SAR-Grenzwert, es erlauben, wieder auf Null abfällt. Der Flipwinkelverlauf 7 innerhalb des Bildgebungsvolumens 1 wird abhängig von der Flussgeschwindigkeit basierend auf experimentellen Daten und/oder theoretischen Überlegungen/Simulationen so gewählt, dass zum einen die erhöhten Magnetresonanzsignale für das einströmende Blut möglichst über das gesamte Bildgebungsvolumen 1 erhalten bleiben, mithin die Zerfallsrate in den Gleichgewichtszustand niedrig genug ist, zum anderen aber eine möglichst gleichbleibende Homogenität in Flussrichtung 3 über das Bildgebungsvolumen 1 resultiert.

Eine Besonderheit ist nach Überschreiten der Frequenzgrenze 9 des Bildgebungsvolumens auf der Einströmseite 4 gegeben, da ersichtlich der lineare Verlauf in einem Abschnitt 10 außerhalb des Bildgebungsvolumens 1 bis zum Flipwinkelwert 0° hin fortgesetzt wird. Damit wird sichergestellt, dass eine maximale Steigung des Flipwinkels (Flipwinkelgradient) nicht überschritten wird. Diese maximal zulässige Steigung ist dabei so gewählt, dass das Produkt aus der Flussgeschwindigkeit des Blutes, der Repetitionszeit und dieser maximalen Steigung im Abschnitt 10 deutlich kleiner als der Nenn-Flipwinkelwert oder aber kleiner als 10° ist. Auf diese Weise wird sichergestellt, dass kein zu starker Flipwinkelunterschied zwischen den Spins in dem Blut besteht, was das korrekte Funktionieren der bSSFP-Magnetresonanzsequenz unterstützt und eine hohe Bildqualität erlaubt. Der Verlauf im Abschnitt 10 wirkt mithin als eine Art Gleichgewichtszustand-Katalysator für einströmendes Blut.

Um nun aus dem Flipwinkelverlauf 7 die Pulsformen des Rampenpulses in der Zeitdomäne zu erhalten, wird eine Fouriertransformation vorgenommen, wonach noch ein Tiefpassfilter, hier ein Hannfilter, auf die Roh-Pulsformen nach der Fouriertransformation angewandt wird. Der Graph 11 zeigt dabei den Absolutanteil 12 der Pulsform in der Zeitdomäne, der Graph 13 den Imaginäranteil 14 der Pulsform in der Zeitdomäne und der Graph 15 den Realanteil 16 der Pulsform in der Zeitdomäne. Die Pulsform wurde dabei gezielt zur Ermöglichung des Einsatzes in der bSSFP-Magnetresonanzsequenz symmetrisch gewählt.

Die so modifizierte bSSFP-Magnetresonanzsequenz lässt sich besonders vorteilhaft zur Aufnahme von 3D-TOF-Magnetresonanzdaten einsetzen.

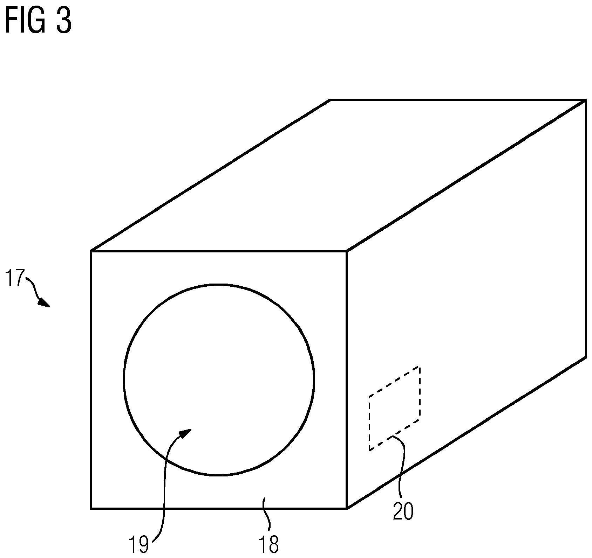

Fig. 3 zeigt schließlich eine Prinzipskizze einer erfindungsgemäßen Magnetresonanzeinrichtung 17, die, wie grundsätzlich bekannt, eine Grundmagneteinheit 18 mit einer Patientenaufnahme 19, in die ein zu untersuchender Patient eingefahren werden kann, aufweist. Die Patientenaufnahme 19 umgebend können, wie grundsätzlich bekannt, eine Hochfrequenzspulenanordnung und eine Gradientenspulenanordnung vorgesehen sein. Diese und andere Komponenten der Magnetresonanzeinrichtung 17 werden mittels einer hier nur angedeuteten Steuereinrichtung 20 der Magnetresonanzeinrichtung 17 angesteuert, welche mithin insbesondere eine Sequenzsteuereinheit zur Steuerung dieser Komponenten zur Realisierung einer beschriebenen bSSFP-Magnetresonanzsequenz aufweisen kann. Ferner kann eine Rampenpulsermittlungseinheit vorgesehen sein, um aus einem vorgegebenen oder selbst zu bestimmenden Flipwinkelverlauf 7, wie beschrieben, Rampenpulse abzuleiten, die dann als regelmäßig auszugebende Hochfrequenzpulse der bSSFP-Magnetresonanzsequenz genutzt werden.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen, der durch die Ansprüche definiert wird.

## Patentansprüche

1. Verfahren zur Aufnahme von Magnetresonanzdaten aus einem aufzunehmenden Bildgebungsvolumen (1) eines Patienten, in dem Flüssigkeit, insbesondere Blut, so bewegt ist, dass die Flüssigkeit auf einer Einströmseite in das Bildgebungsvolumen einströmt und auf einer gegenüberliegenden Ausströmseite aus dem Bildgebungsvolumen ausströmt, wobei die Aufnahme der Magnetresonanzdaten mit einer Magnetresonanzeinrichtung (17) und mittels einer bSSFP-Magnetresonanzsequenz erfolgt, bei der innerhalb des Bildgebungsvolumens (1) befindliche Spins mittels eines Hochfrequenzpulses zyklisch angeregt werden, **dadurch gekennzeichnet, dass** als Hochfrequenzpuls ein einen räumlich innerhalb des Bildgebungsvolumens (1) veränderlichen Flipwinkel der Spins einstellender Rampenpuls verwendet wird, wobei der Flipwinkel auf der Einströmseite (4), von der die Flüssigkeit in das Bildgebungsvolumen (1) einströmt, niedriger gewählt wird als auf der Ausströmseite (5), auf der die Flüssigkeit ausströmt, und monoton ansteigt, wobei eine maximale Steigung des Flipwinkels benachbart zu der die Einströmseite (4) bildenden Grenze des Bildgebungsvolumens (1) durch einen Grenzwert beschränkt wird, der anhand der Repetitionszeit der bSSFP-Magnetresonanzsequenz und der Flussgeschwindigkeit der Flüssigkeit ermittelt wird.

2. Verfahren nach Anspruch 1, **dadurch gekennzeichnet, dass** der Flipwinkelverlauf (7) innerhalb des Bildgebungsvolumens (1) zur Homogenisierung der Magnetresonanzdaten der Flüssigkeit innerhalb des Bildgebungsvolumens (1) und/oder linear steigend von der Einströmseite (4) zu der Ausströmseite (5) und/oder der Rampenpuls (6) zeitlich symmetrisch gewählt wird.

3. Verfahren nach Anspruch 1 oder 2, **dadurch gekennzeichnet, dass** bei der Auslegung des Rampenpulses als Randbedingung eine maximale Belastbarkeit eines Hochfrequenzverstärkers der Magnetresonanzeinrichtung (17) und/oder bei der Auslegung des Gesamtaufnahmevorgangs ein einzuhaltender SAR-Grenzwert berücksichtigt wird.

4. Verfahren nach einem der vorangehenden Ansprüche, **dadurch gekennzeichnet, dass** das Produkt der maximalen Steigung, der Flussgeschwindigkeit und der Repetitionszeit kleiner als der maximal einzustellende Flipwinkelwert und/oder kleiner als ein fester Grenzwert in einem Intervall von 1,5° bis 15° gewählt wird.

5. Verfahren nach einem der vorangehenden Ansprüche, **dadurch gekennzeichnet, dass** der Flipwinkelverlauf (7) auf der Einströmseite (4) außerhalb des Bildgebungsvolumens (1) bei einem Startwert kleiner als 5°, insbesondere 0°, beginnend linear bis zur Ausströmseite (5) des Bildgebungsvolumens (1) ansteigt, insbesondere bei konstanter Steigung.

6. Verfahren nach einem der vorangehenden Ansprüche, **dadurch gekennzeichnet, dass** ein zeitlicher Pulsverlauf des Rampenpulses durch Fouriertransformation des vorgegebenen Flipwinkelverlaufs (7) im Frequenzraum ermittelt wird.

7. Verfahren nach Anspruch 6, **dadurch gekennzeichnet, dass** der Pulsverlauf durch Anwendung eines Tiefpassfilters, insbesondere eines Hannfilters, geglättet wird.

8. Magnetresonanzeinrichtung (17), aufweisend eine zur Durchführung eines Verfahrens nach einem der vorangehenden Ansprüche ausgebildete Steuereinrichtung (20).

9. Computerprogramm, welches die Schritte eines Verfahrens nach einem der vorangehenden Ansprüche 1 bis 7 durchführt, wenn es auf einer Steuereinrichtung (20) einer Magnetresonanzeinrichtung (17) ausgeführt wird.

10. Elektronisch lesbarer Datenträger, auf dem ein Computerprogramm nach Anspruch 9 gespeichert ist.

## Claims

1. Method for capturing magnetic resonance data from an imaging volume (1) to be captured of a patient in which liquid, in particular blood, is moved such that the liquid flows into the imaging volume on an inflow side and flows out of the imaging volume on an opposite outflow side, wherein the capturing of the magnetic resonance data takes place using a magnetic resonance device (17) and by means of a bSSFP magnetic resonance sequence, in which spins located within the imaging volume (1) are cyclically excited by means of a high-frequency pulse, **characterised in that** a ramp pulse is used as the high-frequency pulse which establishes a flip angle of the spins which is spatially variable within the imaging volume (1), wherein the flip angle is selected to be lower on the inflow side (4) from which the liquid flows into the imaging volume (1) than on the outflow side (5) on which the liquid flows out, and increases monotonically, wherein a maximum gradient of the flip angle adjacent to the boundary of the imaging volume (1) forming the inflow side (4) is restricted by a limit value that is determined on the basis of the repetition time of the bSSFP magnetic resonance sequence and the flow velocity of the liquid.

2. Method according to claim 1, **characterised in that** the flip angle profile (7) within the imaging volume (1) is selected to homogenise the magnetic resonance data of the liquid within the imaging volume (1) and/or increasing in linear manner from the inflow side (4) to the outflow side (5) and/or the ramp pulse (6) is selected to be symmetrical over time.

3. Method according to claim 1 or 2, **characterised in that** the parameter taken into account with regard to the design of the ramp pulse is a maximum load rating of a high-frequency amplifier of the magnetic resonance device (17) and/or with regard to the design of the overall capture process is an SAR limit value to be observed.

4. Method according to one of the preceding claims, **characterised in that** the product of the maximum gradient, the flow velocity and the repetition time is selected to be smaller than the maximum flip angle value to be established and/or smaller than a fixed limit value in a range of from 1.5° to 15°.

5. Method according to one of the preceding claims, **characterised in that** the flip angle profile (7) starts on the inflow side (4) outside the imaging volume (1) with a starting value of less than 5°, in particular 0°, and increases in linear manner as far as the outflow side (5) of the imaging volume (1), in particular at a constant gradient.

6. Method according to one of the preceding claims, **characterised in that** the pulse profile of the ramp pulse over time is determined by Fourier transformation of the specified flip angle profile (7) in the frequency space.

7. Method according to claim 6, **characterised in that** the pulse profile is smoothed by application of a low-pass filter, in particular a Hanning filter.

8. Magnetic resonance device (17) having a control device (20) configured to carry out a method according to one of the preceding claims.

9. Computer program which carries out the steps of a method according to one of the preceding claims 1 to 7 when it is executed on a control device (20) of a magnetic resonance device (17).

10. Electronically readable data storage medium on which a computer program according to claim 9 is stored.

## Revendications

1. Procédé d'enregistrement de données de résonnance magnétique dans un volume (1) d'imagerie à enregistrer d'un patient, dans lequel du liquide, notamment du sang, est déplacé de manière à ce que le liquide entre d'un côté d'entrée dans le volume d'imagerie et sorte d'un côté de sortie opposé du volume d'imagerie, dans lequel l'enregistrement des données de résonnance magnétique s'effectue par un dispositif (17) de résonnance magnétique et au moyen d'une séquence de résonnance magnétique bSSFP,
dans lequel on excite cycliquement au moyen d'une impulsion de haute fréquence des spins se trouvant à l'intérieur du volume (1) d'imagerie,
**caractérisé en ce que** l'on utilise comme impulsion de haute fréquence une impulsion en rampe réglant un angle de flip, variable dans l'espace dans le volume (1) d'imagerie, dans lequel on choisit l'angle de flip du côté (4) d'entrée, par lequel le liquide entre dans le volume (1) d'imagerie, plus petit que du côté (5) de sortie, par lequel le liquide sort et il croît de manière monotone, dans lequel on limite une pente maximum de l'angle de flip au voisinage de la limite, formant le côté (4) d'entrée, du volume (1) d'imagerie par une valeur limite, que l'on détermine à l'aide du temps de répétition de la séquence de résonnance magnétique bSSPFP et de la vitesse de flux du liquide.

2. Procédé suivant la revendication 1, **caractérisé en ce que** la courbe (7) de l'angle de flip dans le volume (1) d'imagerie est choisie pour l'homogénéisation des données de résonnance magnétique du liquide dans le volume (1) d'imagerie et/ou pour la croissance linéaire du côté (4) d'entrée au côté (5) de sortie et/ou **en ce que** l'impulsion (6) en rampe est choisie de manière à ce qu'elle soit symétrique dans le temps.

3. Procédé suivant la revendication 1 ou 2, **caractérisé en ce que** pour la conception de l'impulsion en rampe, on prend en compte comme condition aux limites une possibilité de charge maximum d'un amplificateur de haute fréquence du dispositif (17) de résonnance magnétique et/ou, pour la conception du processus d'enregistrement dans son ensemble, une valeur limite SAR à observer.

4. Procédé suivant l'une des revendications précédentes, **caractérisé en ce que** l'on choisit le produit de la pente maximum, de la vitesse du flux et du temps de répétition plus petit que la valeur de l'angle de flip à régler au maximum et/ou plus petit qu'une valeur limite fixe dans un intervalle de 1,5° à 15°.

5. Procédé suivant l'une des revendications précédentes, **caractérisé en ce que** la courbe (7) de l'angle de flip du côté (4) d'entrée a l'extérieur du volume (1) d'imagerie croît pour une valeur initiale plus petite que 5°, notamment de 0°, en commençant linéairement jusqu'au côté (5) de sortie du volume (1) d'imagerie, notamment à pente constante.

6. Procédé suivant l'une des revendications précédentes, **caractérisé en ce que** l'on détermine la courbe en fonction du temps de l'impulsion en rampe par transformation de Fourier dans l'espace fréquentiel de la courbe (7) de l'angle de flip donné à l'avance.

7. Procédé suivant la revendication 6, **caractérisé en ce que** l'on lisse la courbe de l'impulsion par application d'un filtre passe bas, notamment d'un filtre de Hann.

8. Dispositif (17) de résonnance magnétique, comportant un dispositif (20) de commande constitué pour effectuer un procédé suivant l'une des revendications précédentes.

9. Programme d'ordinateur, qui effectue les stades d'un procédé suivant l'une des revendications 1 à 7 précédentes, lorsqu'il est réalisé sur un dispositif (20) de commande d'un dispositif (17) de résonnance magnétique.

10. Support de données déchiffrable électroniquement, sur lequel est mis en mémoire un programme d'ordinateur suivant la revendication 9.
